# FASCICULE DE BREVET EUROPEEN

(11) **EP 4 618 864 B1**
(45) Date de publication et mention de la délivrance du brevet: **25.11.2026**
(21) Numéro de dépôt: 23798289.7
(22) Date de dépôt: 23.10.2023
(51) Int. Cl.: A61B 17/32

(54) **DISPOSITIF DESTINE A REALISER UNE LIBERATION D'UN SYNDROME COMPRESSIF DE LA MAIN**
VORRICHTUNG ZUR FREISETZUNG EINES KOMPRESSIVEN HANDSYNDROMS
DEVICE FOR RELEASING A HAND-COMPRESSION SYNDROME

(30) Priorité: 17.11.2022 EP 22208155
(43) Date de publication de la demande: 24.09.2025
(73) Titulaire: Keri Medical SA, 1228 Plan-les-Ouates (CH)
(72) Inventeur: PRANDI, Bernard, 1226 Thônex (CH); MOTTET, Julie, 74930 Pers-Jussy (FR); APARD, Thomas, 78000 Versailles (FR); COGNET, Jean-Michel, 51140 Jonchery sur Vesle (FR); GARRET, Jérôme, 69006 Lyon (FR)
(74) Mandataire: Micheli & Cie SA
(86) Numéro de dépôt international: PCT/IB2023/060665
(87) Numéro de publication internationale: WO 2024/105477

(56) Documents cités:
- US-A1- 2010 206 099
- US-A1- 2012 016 397
- US-A1- 2017 252 056
- US-A1- 2021 085 361

## Description

La présente invention a pour objet un dispositif destiné à réaliser une libération d'un syndrome compressif de la main, telle la libération du canal carpien.

Le syndrome du canal carpien est un ensemble de signes fonctionnels et physiques liés à la souffrance du nerf médian au niveau du poignet par sa compression et par élévation de la pression au sein du canal carpien. Le canal carpien est la zone de transition entre l'avant-bras et la main. Il est situé dans le talon de la main. Il forme un défilé ostéofibreux inextensible, plus étroit à ce niveau qu'à l'avant-bras ou la main. Le canal carpien est délimité d'une part par les os du carpe qui en constituent le plancher et les côtés, et par le ligament annulaire antérieur du carpe (ou rétinaculum ou ligament rétinaculaire) qui en forme le toit. À l'intérieur de cette structure circulent les tendons fléchisseurs des doigts, le nerf médian et l'artère radiale.

Le syndrome du canal carpien peut résulter de nombreuses maladies, conditions et accidents : œdème, inflammation des tendons, changements hormonaux et mouvements répétés peuvent tous contribuer à l'augmentation de la pression intra canalaire entraînant une compression du nerf médian, responsable du syndrome. Cette compression peut entraîner un engourdissement, des fourmillements dans les doigts, une douleur de la main irradiant au bras et un déficit moteur et peut conduire à la lésion irréversible du nerf.

On dénombre un peu plus de 400 cas pour 100'000 habitants par année aux Etats-Unis, dont un tiers se fait opérer. Le nombre d'opérations pour la France en 2018 était de 125'000. Certains pays reconnaissent le syndrome du canal carpien comme maladie professionnelle.

Quand il y a perte de la sensibilité digitale ou fonte musculaire, le nerf médian est en général sévèrement atteint. Dans les formes légères ou modérées un traitement médical, conservateur, peut être proposé. Dans les formes plus sévères, la chirurgie est préférable. Le traitement chirurgical consiste à sectionner le ligament annulaire antérieur du carpe permettant ainsi la libération du nerf médian des structures qui le compriment. La chirurgie, quelle que soit la technique, à ciel ouvert ou par endoscopie, donne de très bons résultats.

De manière générale, le traitement chirurgical couramment utilisé consiste à réaliser une incision dans le poignet en amont du canal carpien. Une sonde d'échographie ou un endoscope est utilisé pour visualiser le canal et localiser le ligament annulaire antérieur. Un couteau est ensuite inséré dans l'incision pour couper ledit ligament. Certains dispositifs chirurgicaux comprennent un guide inséré avant le couteau et dans lequel ledit couteau peut glisser pour sectionner le ligament. Le guide permet de dilater le canal carpien et de guider le couteau. La coupe du ligament peut se faire de manière antérograde lors de l'insertion du couteau ou de manière rétrograde, le couteau est d'abord inséré dans le canal et la coupe a lieu en sortant le couteau du canal. De tels dispositifs sont connus du document US 2012/016397 A1.

Le risque principal lors de ce traitement chirurgical est d'endommager les tissus mous autour du ligament annulaire antérieur, comme le nerf médian ou l'artère radiale lors de l'insertion et du retrait du guide et/ou du couteau.

Le but de la présente invention est de fournir un dispositif destiné à réaliser une libération d'un syndrome compressif de la main, telle la libération du canal carpien, qui permette une protection entière des tissus mous environnants le ligament à sectionner sans complexifier la technique opératoire.

La présente invention a pour objet un dispositif destiné à réaliser une libération d'un syndrome compressif de la main, telle la libération du canal carpien selon la revendication 1. Des modes de réalisation de l'invention sont décrits dans les revendications dépendantes.
Les figures annexées illustrent schématiquement deux formes d'exécution d'un dispositif destiné à réaliser une libération d'un syndrome compressif de la main selon l'invention.
La figure 1 illustre le dispositif selon une première forme d'exécution de l'invention.
La figure 2 est un agrandissement de l'extrémité distale du guide du dispositif de la figure 1.
La figure 3 est un agrandissement de l'extrémité de coupe du couteau du dispositif de la figure 1.
Les figures 4a à 7 illustrent les étapes du sectionnement du ligament annulaire antérieur pour la libération du canal carpien.

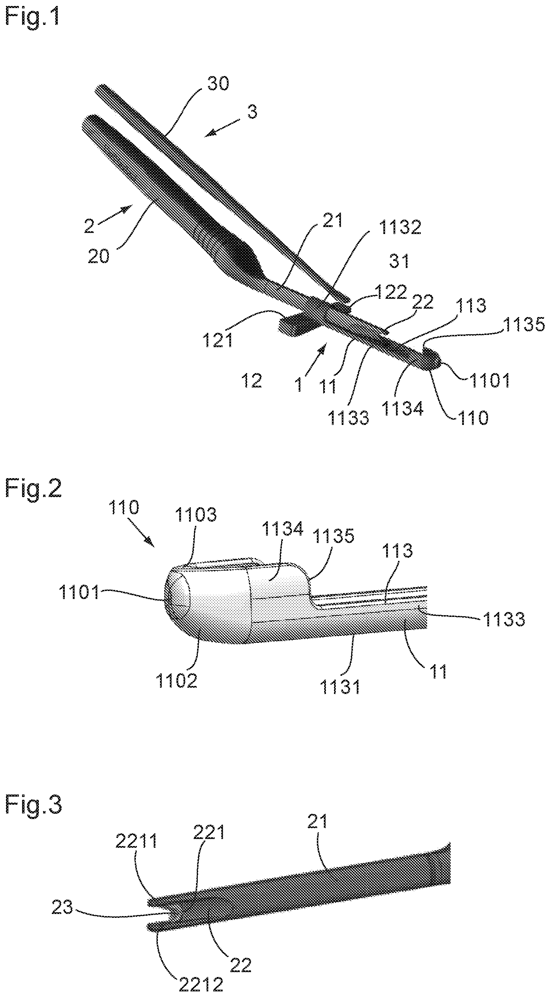

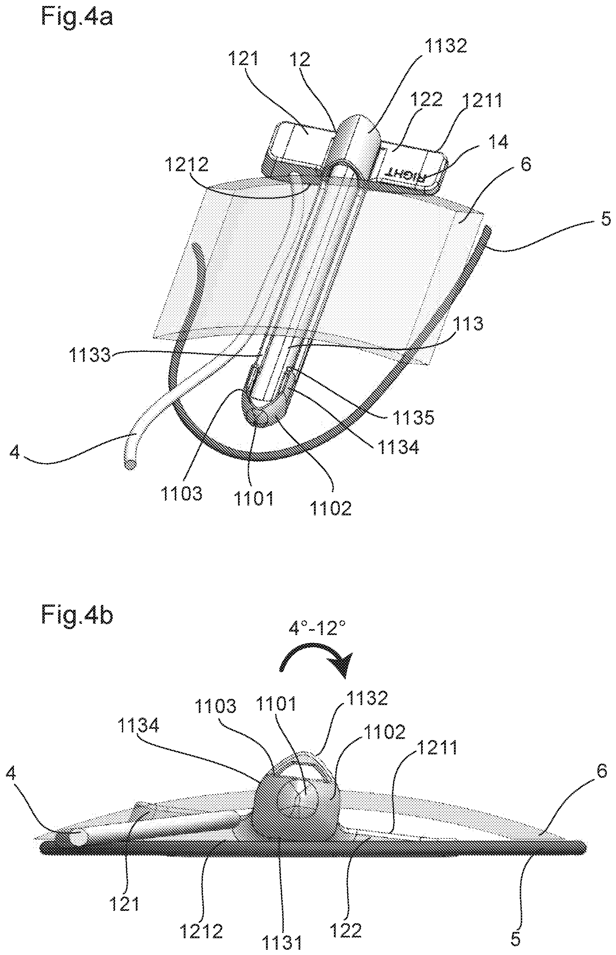

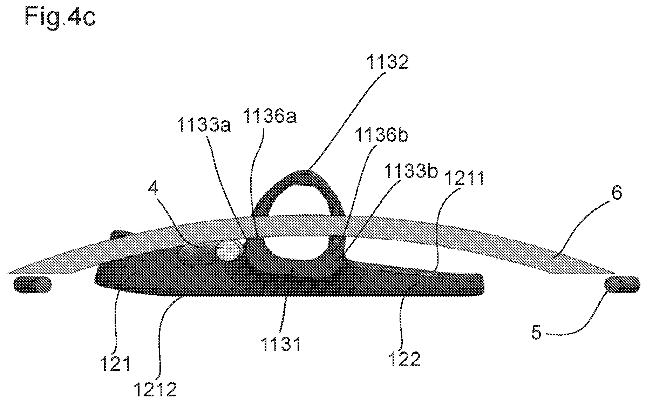

En particulier, les figures 4a et 4b illustrent l'insertion du guide du dispositif de la figure 1 dans le canal carpien vue respectivement en perspective et de face, tandis que la figure 4c est une vue en coupe de la figure 4b selon un axe perpendiculaire à l'axe de la partie longitudinale du guide.

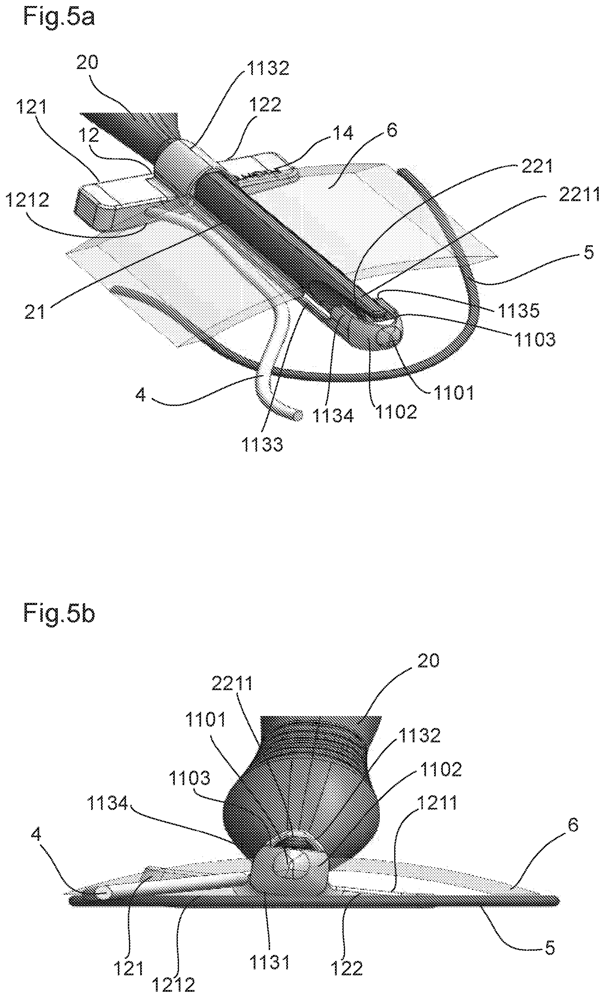

Les figures 5a et 5b illustrent l'insertion du couteau du dispositif de la figure 1 dans le guide pour réaliser la coupe, vue en perspective et de face.

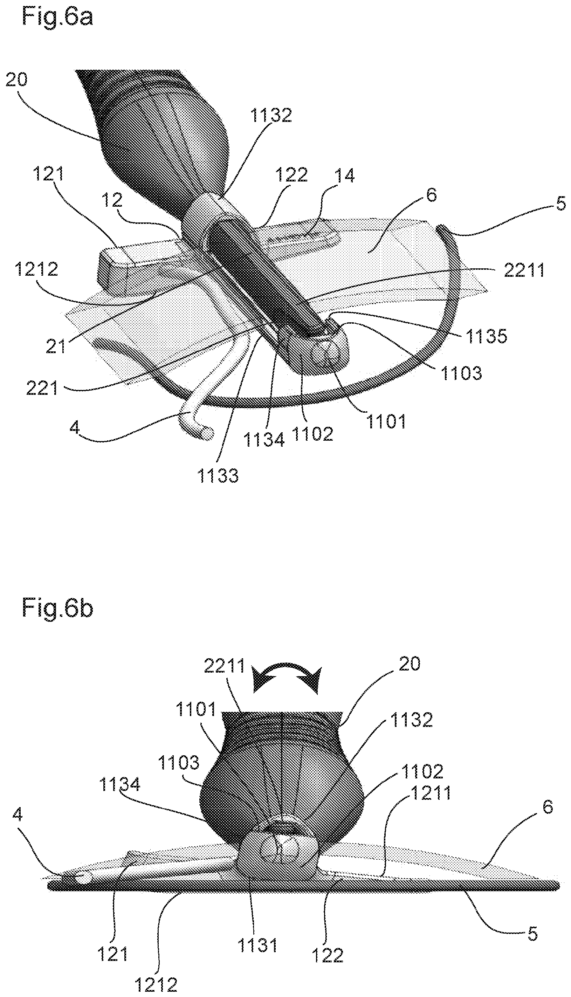

Les figures 6a et 6b illustrent la possibilité pour le praticien d'incliner le couteau dans le guide lors de la coupe, vue respectivement en perspective et de face.

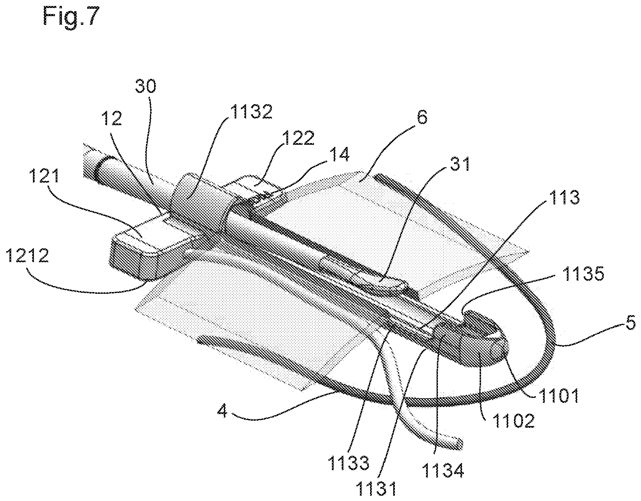

La figure 7 illustre la vérification de la libération complète du ligament par l'insertion de la spatule du dispositif illustré dans la figure 1.

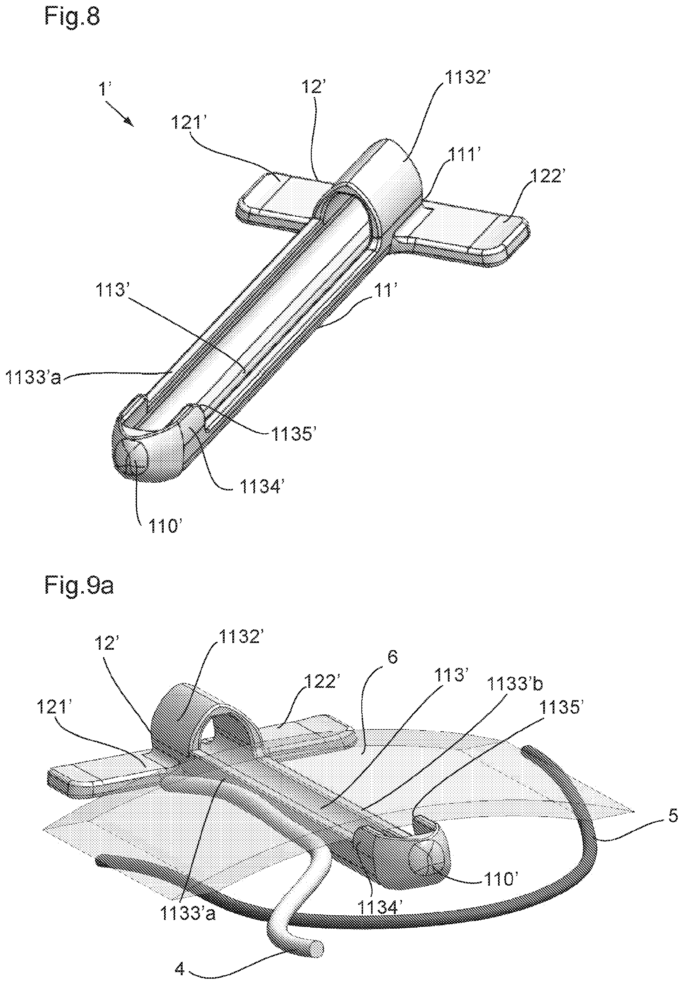

La figure 8 illustre le guide selon une deuxième forme d'exécution de l'invention.

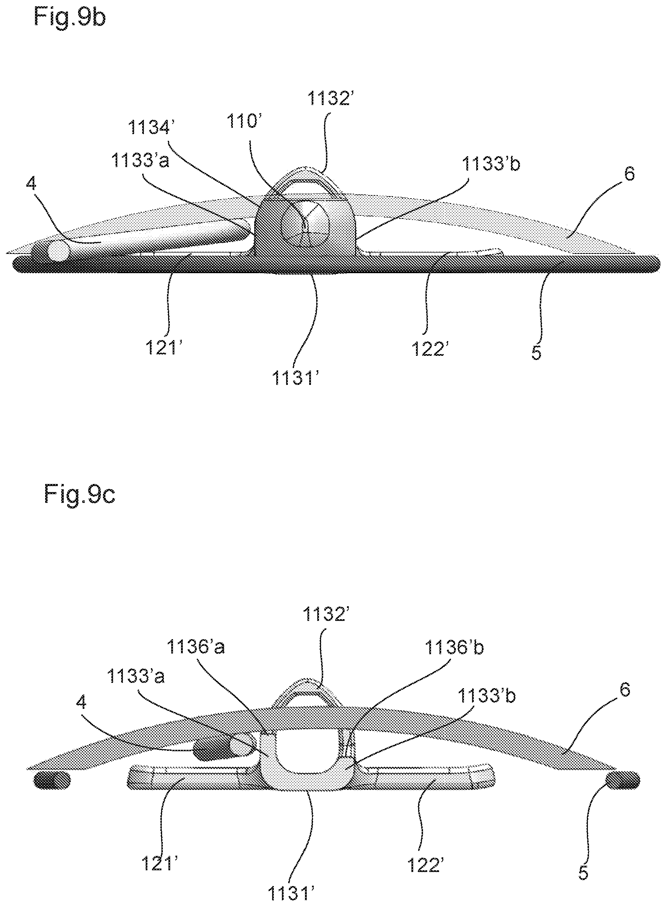

Les figures 9a et 9b illustrent l'insertion du guide de la deuxième forme d'exécution de la figure 8 dans le canal carpien vue respectivement en perspective et de face, tandis que la figure 9c est une vue en coupe de la figure 9b selon un axe perpendiculaire à l'axe de la partie longitudinale du guide.

Dans la première forme d'exécution illustrée aux figures 1 à 7, le dispositif considéré est destiné à réaliser une libération du canal carpien et comprend un couteau 2, un guide 1 et de préférence, une spatule 3.

Le guide 1 comprend une première portion dite longitudinale 11 présentant une extrémité distale 110 et une extrémité proximale. A l'extrémité proximale, le guide 1 comprend une seconde portion transversale 12 formée d'une première et d'une seconde ailettes 121, 122 s'étendant de part et d'autre de la première portion longitudinale 11.

Au moins un canal 113 est formé dans la portion longitudinale 11 du guide 1 et est destiné à recevoir le couteau 2 lors de la procédure de libération du canal carpien. Le canal 113 est fermé vers le bas par son fond 1131 sur toute la longueur de la première portion 11 du guide 1. Le canal 113 est fermé vers le haut et sur les côtés à la hauteur de l'extrémité proximale de la portion longitudinale 11 du guide 1 sur une partie qu'on appellera tunnel d'entrée 1132 du canal 113. Le canal 113 est ouvert vers le haut à la suite du tunnel d'entrée 1132 jusqu'à l'extrémité distale 110.

La hauteur des parois latérales du canal 113 varie sur sa longueur : ledit canal 113 présente des parois latérales hautes 1134 à l'extrémité distale 110 du guide 1 et des parois latérales plus basses 1133a, 1133b entre le tunnel d'entrée 1132 et ladite extrémité distale 110. Le décrochement 1135 formé à la jointure entre les parois latérales hautes 1134 et les parois latérales basses 1133a, 1133b du canal 113 permet de crocheter le ligament 6 devant être sectionner et de le bloquer dans le guide 1 une fois celui-ci en position. Le décrochement 1135 est conformé pour être atraumatique et ne pas endommager les tissus lors du retrait du guide 1.

Dans la première forme d'exécution, les parois latérales basses 1133a, 1133b présentent la même hauteur des deux côtés du canal 113. Ainsi, le plan passant par les sommets 1136a, 1136b des parois latérales basses 1133a, 1133b est parallèle au plan du fond 1131 du canal 113.

En variante, la portion longitudinale 11 du guide 1 pourrait comprendre un second canal destiné à recevoir un instrument comme un endoscope ou un arthroscope et s'étendant parallèlement au premier canal 113. De préférence, les canaux destinés à recevoir le couteau et l'instrument sont alors séparés par une paroi pour éviter toute interférence.

L'extrémité distale 110 de la portion longitudinale 11 du guide 1 présente essentiellement la forme d'une ogive tronquée sur le dessus. En particulier, l'extrémité distale 110, dont un agrandissement est visible sur la figure 2, comprend une pointe 1101 arrondie, légèrement pointue pour faciliter l'insertion du guide, tout en étant atraumatique (pas piquante) pour préserver les tissus lors de l'insertion. La partie 1102 entre la pointe 1101 et le fond 1131 et les parois latérales hautes 1134 du canal 113 est courbée, toujours pour une insertion facile du guide dans le canal carpien. La partie supérieure 1103 de l'extrémité distale 110 est tronquée et plate pour permettre son identification par ultrasons. Cette partie supérieure plate 1103 rejoint les parois latérales hautes 1134 du canal 113. De préférence, la pointe 1101 est à une hauteur minimale par rapport au fond 1131 du canal 113 pour garantir que les tissus ne seront pas endommagés lors de l'insertion du guide 1 dans le canal carpien et en particulier pour éviter que le guide 1 n'attrape l'arche de l'artère radiale 5. Comme on le verra plus loin lors de la description détaillée de l'utilisation du dispositif, l'extrémité distale 110 est également conformée pour servir de butée au couteau 2 lors de la translation de celui-ci dans le canal 113.

La portion transversale 12 et les ailettes 121 et 122 servent à manier le guide pour son insertion et son retrait dans le canal carpien. Selon la première forme d'exécution de l'invention, les ailettes 121, 122 sont agencées de sorte qu'il existe un angle aigu non nul, de préférence de 4° à 12° entre le plan directeur des ailettes 121, 122 et le plan du fond 1131 du canal 113 ou le plan des sommets 1136a, 1136b des parois latérales basses 1133a, 1133b. Ainsi, lorsque lesdites ailettes 121, 122 reposent sur une surface essentiellement plane comme la face antérieure de l'avant-bras sur laquelle repose les ailettes 121, 122 lors d'un traitement pour la libération du canal carpien, la portion longitudinale 11 et le fond 1131 du canal 113 sont inclinés de 4° à 12° par rapport au plan de la face antérieure de l'avant-bras. Pour ce faire, les ailettes 121, 122 présentent une face supérieure 1211 essentiellement parallèle au fond 1131 du canal 113 et une face inférieure 1212 dont le plan forme un angle compris entre 4° et 12° avec le plan du fond 1131 du canal 113 du la portion longitudinale 11 du guide 1 ou le plan des sommets 1136a, 1136b des parois latérales basses 1133a, 1133b. La face inférieure 1212 est destinée à venir en appui sur la face antérieure de l'avant-bras et de par son angulation déterminer l'inclinaison de la portion longitudinale 11 et du canal 113.

De manière générale, il est souhaitable que le fond 1131 du canal 113 ou le plan passant par les sommets 1136a, 1136b des parois latérales basses 1133a, 1133b présente une inclinaison en direction du petit doigt lorsque le guide 1 est inséré dans sa position de service dans le canal carpien depuis le poignet en direction des doigts. C'est à dire que l'angle aigu non nul, de préférence de 4° à 12°, entre le plan passant par les sommets 1136a, 1136b des parois latérales basses 1133a, 1133b et le plan de la face inférieure 1212 de la seconde portion transversale 12 est ouvert en direction du nerf médian 4 en position de service du dispositif. Ainsi, selon le poignet à traiter, gauche ou droite, il faudra un guide 1 approprié. Le guide 1 peut donc par exemple comprendre une indication 14 sur sa partie transversale 12 permettant d'identifier s'il s'agit d'un guide pour la main gauche ou pour la main droite. Dans la forme d'exécution illustrée, cette indication 14 est le mot « right » gravé sur l'une des ailettes 122.

Un autre élément du dispositif selon l'invention est le couteau 2. Comme illustré sur la figure 1, le couteau 2 comprend un manche 20 et une partie active 21. La partie active 21 forme un angle de 20° avec le manche 20. La partie active 21 se termine par une extrémité de coupe 22 qui porte la lame de coupe 23 destinée à sectionner le ligament annulaire antérieur 6. La partie active 21 est destinée à être glissée dans le canal 113 de la portion longitudinale 11 du guide 1.

Dans la forme d'exécution illustrée, le couteau 2 et son extrémité de coupe 22 sont agencés pour réaliser une coupe antérograde, c'est-à-dire lors de l'insertion du couteau dans le canal carpien. En variante, le couteau 2 et son extrémité de coupe 22 pourrait être agencés pour réaliser une coupe rétrograde, c'est-à-dire lors du retrait du couteau du canal carpien.

De préférence, l'extrémité de coupe 22 présente une encoche 221 dans laquelle est retenue la lame de coupe 23. L'encoche 221 forme une languette supérieure 2211 et une languette inférieure 2212 qui protègent la lame de coupe 23 et évitent que celle-ci n'entre en contact avec les tissus environnants le ligament annulaire antérieur 6. De préférence, la lame de coupe 23 est surmoulée dans l'extrémité de coupe 22 dans l'encoche 221.

Enfin, le dispositif selon la forme d'exécution illustrée comprend de préférence une spatule 3. La spatule 3 est un instrument long et effilé comprenant un manche 30 et une extrémité active 31 de forme plate et arrondie. Comme on le verra plus loin, la spatule 3 est destinée dans un premier temps à préparer l'insertion du guide 1 dans le canal carpien et dans un second temps, à vérifier la libération complète du ligament 6. Pour ce faire, la spatule 3 est également adaptée à coulisser dans le canal 113 de la portion longitudinale 11 du guide 1.

Dans la suite, la technique opératoire permettant la libération du canalcarpien à l'aide d'un dispositif selon la forme d'exécution de l'invention ci-dessus est décrite en détails en référence aux figures 4a à 7.

Dans l'exemple illustré aux figures, il s'agit de libérer le canal carpien du poignet droit d'un patient.

La première étape consiste à réaliser une incision d'environ 1 cm au pli de flexion du poignet sur le palmaris longus (face antérieure de l'avant-bras). Après avoir exposé et incisé le carpi volare (partie précédant le début du canal carpien), la spatule 3 est insérée sous le ligament annulaire antérieur du carpe 6 pour vérifier qu'il n'y a pas de rameau nerveux transligamentaire (zone de dépression dans le ligament) et pour préparer l'insertion du guide. De préférence, la spatule 3 est ensuite glissée au-dessus du ligament 6 pour faciliter l'avancée ultérieure du couteau 2.

Une sonde échographique est ensuite utilisée pour localiser la zone propice à l'introduction du guide 1 et du couteau 2 sans risquer d'endommager les structures à risque dans et autour du canal carpien comme le nerf médian 4 et l'artère radiale 5.

La guide 1 est ensuite inséré dans cette zone propice sous le ligament annulaire antérieur du carpe 6. Pour ce faire, le praticien empoigne le guide 1 par les ailettes 121, 122 et fait glisser la portion longitudinale 11 du guide 1 dans l'incision. La forme en ogive de l'extrémité distale 110 du guide 1 permet de garantir qu'aucun tissu mou environnant n'est endommagé pendant l'insertion du guide 1. De plus, avec sa pointe 1101 arrondie mais non piquante et haute, ladite extrémité distale 110 ne risque pas d'endommager l'artère radiale 5 qui fait son arche autour du canal carpien. La figure 5b illustre en particulier le guide 1 dans sa position finale dans le canal carpien sous le ligament annulaire antérieur 6. La pointe 1101 passe au-dessus de l'artère radiale 5 et celle-ci a éventuellement pu être repoussée par le guide 1 notamment par la partie 1102 courbée de l'extrémité distale 110.

Le guide 1 est inséré dans le canal carpien jusqu'à sa position finale, c'est-à-dire jusqu'à ce que l'extrémité distale 110 en forme d'ogive passe le ligament 6 et que ledit ligament 6 passe le décrochement 1135 du guide 1, cela correspond en général à une position dans laquelle l'entier de sa portion longitudinale 11 est dans le canal carpien ou encore dans laquelle le tunnel d'entrée 1132 vient en butée contre l'avant-bras. Dans cette position finale, l'extrémité distale 110 est dégagée du ligament 6 qui est crocheté et bloqué dans le canal 113. La figure 4b illustre en particulier la façon dont le décrochement 1135 du canal 113 permet de crocheter le ligament 6 et le bloquer dans le guide 1.

L'insertion du guide 1 dans le canal carpien se fait dans un plan essentiellement parallèle au plan de la face antérieure de l'avant-bras. Selon l'invention, le guide est agencé pour que lors de cette insertion la portion longitudinale 11 et le canal 113 sont inclinés de sorte que le fond 1131 dudit canal n'est pas parallèle au plan de la face antérieure de l'avant-bras mais est incliné de 4° à 12° par rapport audit plan, l'angle étant ouvert en direction du nerf médiant 4 et du petit doigt. Cette inclinaison permet de présenter ledit fond 1131 au nerf médian 4 qui va être écarté de la zone de coupe lors de l'insertion du guide, réduisant ainsi les risques de dommages causés à ce nerf 4 lors de la coupe du ligament 6. Les figures 4b et 4c en particulier illustrent le guide 1 selon la première forme d'exécution, en place dans sa position finale de service (extrémité distale 110 dégagée du ligament 6 et ligament 6 crocheté dans le décrochement 1135). Dans cette position finale de service, l'inclinaison du canal 113 fait que les parties du guide 1 les plus proches du nerf médian 4 sont les parois latérales basses 1133 et le fond 1131 du canal 113, parties qui empêchent le couteau 2 de venir en contact avec ledit nerf médian 4 lorsque ledit couteau 2 coulisse dans le canal 113 du guide 1.

Une fois le guide 1 inséré dans sa position finale de service, le praticien peut relâcher les ailettes 121, 122. Dans la première forme d'exécution, de par leur agencement et notamment leur face inférieure 1212, le guide 1 et sa portion longitudinale 11 gardent leur inclinaison de 4° à 12° lorsque ladite face inférieure 1212 vient s'appuyer contre la face antérieure de l'avant-bras.

Comme illustré sur les figures 4a à 4c, lors de l'insertion du guide 1, la portion longitudinale 11 du guide 1, et en particulier, le fond 1131 du canal 113 écartent et protègent le nerf médian 4 qui s'éloigne du canal 113 et de la zone de coupe, réduisant ainsi les risques de dommages causés à ce nerf 4 lors de la coupe du ligament 6. Le nerf médian 4 vient se loger dans l'angle aigu non nul formé entre le plan de la surface inférieure 1212 de la seconde portion transversale 12 et le plan passant par les sommets 1136a, 1136b des parois latérales basses 1133a, 1133b.

En variante, le praticien pourrait insérer le guide 1 dans le canal carpien en le faisant glisser sur le fond 1131 du canal 113. Même dans cette situation, grâce à l'agencement de ailettes 121, 122 de la première forme d'exécution, en relâchant le guide 1, la face inférieure 1212 desdites ailettes vient s'appuyer contre la face antérieure de l'avant-bras et fait pivoter le canal 113 de 4° à 12° en direction du petit doigt. Ce pivotement écarte le nerf médian 4 qui fait désormais face au fond 1131 et aux parois latérales 1133, 1134 du canal 113 et est ainsi protégé.

Dans cette position inclinée de service, le guide 1 est prêt à recevoir le couteau 2 pour la découpe du ligament 6. Avant cela, la sonde d'échographie est à nouveau utilisée pour confirmer que le guide est bien positionné et que la coupe peut avoir lieu sans risque pour les structures environnantes du ligament 6 (nerf médian 4, arcade palmaire). Comme indiqué plus haut, la forme en ogive tronquée de l'extrémité distale 110 du guide 1 et sa partie supérieure 1103 plate permettent de la repérer facilement par échographie.

Le couteau 2 est ensuite introduit dans le guide 1 à travers le tunnel d'entrée 1132 pour coulisser dans le canal 113 et couper le ligament 6 (figures 5a et 5b). Pendant l'insertion du couteau 2, le praticien maintient les ailettes 121, 122 du guide 1. Si le praticien estime qu'il a besoin de plus d'inclinaison du guide, il peut maintenir les ailettes 121, 122 sous contrainte pour obtenir une inclinaison du fond 1131 par rapport au plan de la face antérieure de l'avant-bras supérieure à 12°.

Le couteau 2 est introduit jusqu'à ce son extrémité de coupe 22 vienne en butée dans l'extrémité distale 110 de la portion longitudinale 11 du guide 1, ladite extrémité distale 110 étant conformée pour arrêter toute translation du couteau 2 au-delà de ce point et ainsi protéger l'arche de l'artère radiale 5. En variante, le manche 20 du couteau 2 peut également comprendre une marque indiquant que le couteau 2 ne doit pas être inséré plus avant ou une butée agencée pour coopérer avec le tunnel d'entrée 1132 de l'extrémité proximale 111 de la portion longitudinale 11 du guide 1.

Durant l'insertion du couteau 2, sa partie active 21 et en particulier la languette inférieure 2212 est maintenue en contact avec le fond 1131 du canal 113 du guide 1 pour garantir une coupe sûre. La languette supérieure 2211 du couteau 2 permet de protéger l'aponévrose palmaire superficielle durant la coupe et fermer l'ogive de l'extrémité distale 110 du guide 1 (figure 5a).

De préférence, le couteau 2 et notamment sa partie active 21 et le canal 113 du guide 1 sont agencés pour permettre une rotation dans un ou deux sens de la partie active 21 du couteau 2 dans le canal 113. De préférence, des moyens sont prévus sur la partie active 21 du couteau 2 et/ou dans le canal 113 pour limiter la rotation du couteau 2 par rapport audit canal 113, de préférence à des angles entre 8° et 15°. Ces moyens peuvent consister en un système de forme et contreforme ou en un système de butée. Cette rotation possible de la partie active 21 du couteau 2 dans le canal 113 a plusieurs avantages. D'une part, elle permet une amorce de la coupe plus aisée. Elle permet d'autre part de débloquer le couteau 2 en cas de coupe plus dure. Ensuite, en pivotant le couteau 2 dans le sens opposé à l'inclinaison du canal 113, l'entraxe entre la languette supérieure 2211 de la partie active 21 du couteau 2 et le fond 1131 du canal 113 est augmenté, ce qui augmente l'épaisseur du ligament 6 admissible dans l'encoche 221 de l'extrémité de coupe 22 du couteau 2. La figure 6b illustre cette contre-rotation du couteau 2 par rapport au canal 113 du guide 1.

Lorsque l'extrémité de coupe 22 du couteau 2 est arrivée en butée dans l'extrémité distale 110 du guide 1, le ligament annulaire antérieur 6 a été coupé. Le couteau 2 peut alors être retiré en maintenant le guide en place, par exemple par ses ailettes 121, 122.

De préférence, pour confirmer la libération complète du ligament 6, la spatule 3 est ensuite insérée dans le guide 1 en la glissant dans le canal 113. L'extrémité distale 110 du guide 1 est conformée pour servir de butée en translation dans le canal 113 à l'extrémité active 31 de la spatule 3 et empêcher ladite extrémité active 31 de causer des dommages à l'artère radiale 5. La spatule 3 est ensuite retirée, avant le retrait du guide 1. La libération du canal carpien est ainsi accomplie en évitant tout dommage aux tissus et structures environnantes sans pour autant complexifier la procédure.

Les figures 8 à 9c illustrent une seconde forme d'exécution du dispositif selon l'invention. Dans cette forme d'exécution, le couteau 2 et la spatule 3 sont identiques à ceux décrits en référence à la première forme d'exécution. Seul le guide change.

De manière similaire à la première forme d'exécution, le guide 1' illustré à la figure 8 comprend une première portion dite longitudinale 11' présentant une extrémité distale 110' et une extrémité proximale. A l'extrémité proximale, le guide 1' comprend une seconde portion transversale 12' formée d'une première et d'une seconde ailettes 121', 122' s'étendant de part et d'autre de la première portion longitudinale 11'.

Au moins un canal 113' est formé dans la portion longitudinale 11' du guide 1' et est destiné à recevoir le couteau 2 lors de la procédure de libération du canal carpien. Le canal 113' est fermé vers le bas par son fond 1131' sur toute la longueur de la première portion 11' du guide 1'. Le canal 113' est fermé vers le haut et sur les côtés à la hauteur de l'extrémité proximale de la portion longitudinale 11' du guide 1' sur une partie qu'on appellera tunnel d'entrée 1132' du canal 113'. Le canal 113' ouvert vers le haut à la suite du tunnel d'entrée 1132' jusqu'à l'extrémité distale 110'.

La hauteur des parois latérales du canal 113' varie sur sa longueur : ledit canal 113' présente des parois latérales hautes 1134' à l'extrémité distale 110' du guide 1' et des parois latérales plus basses 1133' entre le tunnel d'entrée 1132' et ladite extrémité distale 110'. Un décrochement 1135' est formé à la jointure entre les parois latérales hautes 1134' et les parois latérales basses 1133' du canal 113'. Ce décrochement 1135' permet de crocheter le ligament 6 devant être sectionné et de le bloquer dans le guide 1' une fois celui-ci en position. Le décrochement 1135' est conformé pour être atraumatique et ne pas endommager les tissus lors du retrait du guide 1.

Selon la forme d'exécution illustrée aux figures 8 à 9b, les parois latérales basses 1133' ne présentent pas la même hauteur de part et d'autre du canal 113' : une première paroi latérale basse 1133', la paroi basse gauche 1133'a dans les figures, est plus haute que l'autre paroi latérale basse 1133', la paroi basse droite 1133'b. Les parois latérales basses 1133' sont agencées et en particulier présentent une différence de hauteur telle qu'un plan passant par le sommet respectif 1136'a, 1136'b de chacune des dites parois latérales basses 1133a', 1133'b forme un angle aigu non nul, de préférence compris entre 4° et 12° avec le plan du fond 1131' du canal 113' de la portion longitudinale 11' du guide 1' ou avec le plan de la seconde portion transversale 12'. Ainsi, lorsque le guide est posé sur une surface plane, son fond 1131' parallèle à ladite surface plane, le plan passant par les sommets 1136'a, 1136'b des parois latérales basses 1133'a et 1133'b forme un angle aigu non nul de préférence compris entre 4° et 12° avec ladite surface plane.

De manière générale, il est souhaitable que le plan passant par les sommets 1136'a, 1136'b des parois latérales basses 1133'a, 1133'b présente une inclinaison en direction du petit doigt lorsque le guide 1' est inséré dans le canal carpien depuis le poignet en direction des doigts. C'est à dire que l'angle aigu non nul de préférence de 4° à 12° entre le plan passant par les sommets 1136'a, 1136'b des parois latérales basses 1133'a, 1133'b et le plan de la seconde portion transversale 12' est ouvert en direction du nerf médian 4 en position de service du dispositif. Ainsi, selon le poignet à traiter, gauche ou droite, il faudra un guide 1' approprié : dans les figures, la paroi basse gauche 1133'a est plus haute que la paroi basse droite 1133'b, ce qui implique que le guide 1' est adapté pour un poignet droit. De manière générale, la première paroi latérale basse 1133'a immédiatement adjacente au nerf médian 4 dans la position de service du guide 1' est plus haute que la seconde paroi latérale basse 1133'b.

Les autres caractéristiques du guide 1' et en particulier son extrémité distale 110' en forme d'ogive sont en tout point similaires à ce qui a été décrit ci-dessus en référence à la première forme d'exécution.

Comme dans la première forme d'exécution, la portion transversale 12' et les ailettes 121' et 122' servent à manier le guide 1' pour son insertion et son retrait dans le canal carpien.

Pour insérer le guide 1' selon cette variante sous le ligament annulaire antérieur du carpe 6, le praticien empoigne le guide 1' par les ailettes 121', 122' et fait glisser la portion longitudinale 11' du guide 1' dans l'incision. La forme en ogive de l'extrémité distale 110' du guide 1' permet de garantir qu'aucun tissu mou environnant n'est endommagé pendant l'insertion du guide 1. Le guide 1' est inséré dans le canal carpien jusqu'à sa position finale, c'est-à-dire jusqu'à ce que l'extrémité distale 110' en forme d'ogive passe le ligament 6 et que ledit ligament 6 passe le décrochement 1135' du guide 1' ; cela correspond en général à une position dans laquelle l'entier de sa portion longitudinale 11' est dans le canal carpien ou encore dans laquelle le tunnel d'entrée 1132' vient en butée contre l'avant-bras. Dans cette position finale, l'extrémité distale 110' est dégagée du ligament 6 qui est crocheté et bloqué dans le canal 113'par le décrochement 1135' comme illustré sur la figure 9a.

L'insertion du guide 1' dans le canal carpien se fait dans un plan essentiellement parallèle au plan de la face antérieure de l'avant-bras. La différence de hauteur entre la paroi basse gauche 1133'a et la paroi basse droite 1133'b, permet de présenter la paroi basse gauche 1133'a plus haute au nerf médian 4 qui va être écarté de la zone de coupe lors de l'insertion du guide, réduisant ainsi les risques de dommages causés à ce nerf 4 lors de la coupe du ligament 6.

Le dispositif destiné à réaliser une libération du canal carpien dans les deux alternatives ci-dessus a été décrit à titre d'exemple uniquement.

En particulier, le dispositif illustré est approprié pour une libération du canal carpien de la main droite. Comme indiqué ci-dessus, un dispositif approprié pour la main gauche est tout à fait similaire, le guide 1 selon la première forme d'exécution étant simplement agencé pour produire une inclinaison du canal 113 dans le sens opposé pour protéger le nerf médian tandis que le guide 1' selon la seconde forme d'exécution présentera soit la paroi basse gauche 1133'a plus basse que la paroi basse droite 1133'b. En d'autres termes, le plan passant par le sommet respectif 1136'a, 1136'b de chacune des parois latérales basse 1133a', 1133'b forme un angle aigu non nul, de préférence compris entre 4° et 12° avec le plan du fond 1131' du canal 113' de la portion longitudinale 11' du guide 1' et cet angle est ouvert en direction du nerf médian 4 en position finale de service du guide 1'. Le couteau et la spatule ne changent pas. En variante, le dispositif pourrait comprendre un couteau, une spatule et deux guides selon l'une des première ou seconde formes d'exécution, un pour le poignet droit et un pour le poignet gauche.

De préférence, le dispositif est à usage unique et ses composants sont réalisés dans tout matériau approprié à l'usage médical.

Dans les formes d'exécution illustrées, le couteau 2 est agencé pour réaliser une coupe antérograde. En variante, le couteau pourrait être agencé pour réaliser une coupe rétrograde. Dans ce cas, le couteau est introduit dans le canal 113 jusqu'à venir en butée contre l'extrémité distale 110 du guide 1. Avec la forme de ladite extrémité distale et du décrochement 1135, dans cette position de butée l'extrémité de coupe du couteau rétrograde est garantie d'avoir dépassé le ligament 6 qui est crocheté dans le décrochement 1135. Il est alors possible de pivoter le couteau vers le haut du canal 113 pour engager l'extrémité de coupe rétrograde dans le ligament et amorcer la coupe. La rotation du couteau par rapport au canal 113 selon l'axe longitudinal dudit couteau présente les mêmes avantages que dans le cas d'une découpe antérograde : amorce de coupe facilitée, déblocage possible en cas de coupe dure, possible d'admettre des ligaments plus épais en pivotant le couteau dans le sens opposé à l'inclinaison du guide. Il en va de même avec un guide selon la seconde forme d'exécution de l'invention.

En variante, la portion longitudinale du guide pourrait comprendre un second canal distinct du premier, séparé du premier par une cloison et destiné à recevoir un instrument comme une caméra endoscopique.

En variante, le dispositif pourrait ne pas comprendre de spatule.

En variante, le dispositif pourrait ne pas comprendre de décrochement 1135.

De manière générale, le dispositif destiné à réaliser une libération d'un syndrome compressif de la main comprend :
- Un couteau comprenant un manche et une extrémité de coupe recevant des moyens de coupe ; et
- Un guide comprenant une portion longitudinale avec une extrémité distale et une extrémité proximale et présentant au moins un canal dans lequel peut coulisser le couteau, ledit canal étant délimité par une première et une seconde parois latérales dont les sommets définissent un premier plan ;
- Le guide comprend en outre une seconde portion transversale définissant un second plan.

Selon l'invention, le guide est agencé de sorte que, lors de l'insertion du guide et/ou une fois inséré dans sa position finale de service dans la zone à traiter de la main, le guide est dans une position dans laquelle le premier plan passant par les sommets des première et seconde parois latérales du canal forme un angle aigu non nul, de préférence compris entre 4° et 12°, avec le second plan défini par la seconde portion transversale.

Ainsi, dans la position finale du guide et/ou lors de son insertion, le premier plan passant par les sommets des première et second parois latérales du canal est incliné dans le canal carpien en direction du nerf médian ce qui permet d'écarter le nerf médian dudit canal lors de l'insertion du guide et de protéger ledit nerf médian en lui présentant le fond du canal et/ou une des première ou seconde parois latérales.

Selon une première alternative de l'invention, la portion transversale est destinée à venir en appui sur une face essentiellement plane du bras ou de la main et est agencée de sorte que lorsque la portion transversale est en appui sur un second plan, il existe un angle aigu non nul, de préférence compris entre 4° et 12°, entre ledit second plan et le premier plan. Dans cette alternative, le premier plan est parallèle au plan du fond du canal.

Ainsi, dans cette première alternative, lorsque la portion transversale du guide vient en appui sur la face antérieure de l'avant-bras, le canal est incliné dans le canal carpien ce qui permet d'écarter le nerf médian dudit canal lors de l'insertion du guide et de protéger ledit nerf médian en lui présentant le fond du canal.

Selon une seconde alternative, les première et seconde parois latérales sont agencées de sorte que la première desdites parois latérales présente, sur la majeure partie de la longueur de la portion longitudinale du guide, une hauteur supérieure à la seconde paroi latérale. La différence de hauteur entre les première et seconde parois latérales est telle que le premier plan passant par le sommet de chacune desdites parois latérales forme un angle aigu non nul compris entre 4° et 12° avec le second plan de la seconde portion transversale qui est parallèle au fond du canal de la portion longitudinale du guide dans cette alternative. En d'autres termes, cette différence de hauteur permet au premier plan défini par les sommets des première et second parois latérale de former un angle aigu non nul, de préférence compris entre 4° et 12°, avec le plan défini par la face antérieure de l'avant-bras de la main à traiter, lors de l'insertion dudit guide dans la zone à traiter.

Ainsi, dans cette seconde alternative également, lors de l'insertion du guide et/ou dans sa position finale, la différence de hauteur entre les première et seconde parois latérales du canal et l'inclinaison du premier plan défini par le sommet desdites parois latérales permet d'écarter le nerf médian dudit canal et de protéger ledit nerf médian en lui présentant la première paroi latérale la plus haute.

Le dispositif selon l'invention peut comprendre en outre les caractéristiques optionnelles suivantes quelle que soit l'alternative utilisée pour le guide.

De préférence, l'extrémité distale de la portion longitudinale du guide présente essentiellement une forme d'ogive avec une pointe arrondie et une partie courbée entre le fond, les parois latérales du canal et ladite pointe. De préférence, la distance entre la pointe et le fond du canal est la plus grande possible. En outre, ladite extrémité distale est agencée pour empêcher la translation du couteau et de son extrémité de coupe hors du canal. Ainsi, l'extrémité distale est conformée pour préserver les tissus lors de l'insertion du guide dans la zone de coupe et lors de l'insertion du couteau dans le guide en servant de butée à ce dernier.

De préférence, l'extrémité distale a une forme d'ogive tronquée avec une partie supérieure plate pour permettre son identification par ultrasons.

De préférence, le couteau comprend une languette inférieure destinée à coulisser contre le fond du canal et une languette supérieure destinée à faciliter l'insertion du couteau et à protéger les tissus environnants la zone de coupe, une encoche entre lesdites languettes reçoit une lame de coupe et forme l'extrémité de coupe du couteau. La languette supérieure est agencée pour venir fermer l'extrémité distale du guide lorsque l'extrémité de coupe du couteau est en butée dans ladite extrémité distale. Les tissus environnants sont donc protégés à 360° des moyens de coupe.

De préférence, le couteau et le guide sont agencés pour que ledit couteau puisse pivoter dans le canal de la portion longitudinale du guide dans un sens ou dans l'autre, d'un angle compris de préférence entre 8° et 15°. Cette rotation permet de faciliter l'amorce de coupe et débloquer le couteau en cas de coupe dur. De plus, selon le sens de rotation du couteau par rapport au sens d'inclinaison du guide selon la première alternative, il est possible d'augmenter l'épaisseur du ligament recevable dans l'extrémité de coupe.

De préférence, le dispositif comprend en outre une spatule comportant un manche et une extrémité active plate et arrondie. La spatule est agencée pour pouvoir coulisser dans le canal du guide jusqu'à venir en butée contre l'extrémité distale de la portion longitudinale du guide, l'empêchant ainsi de sortir du canal et protégeant les tissus environnant de la zone de coupe. La spatule utilisée seule permet de préparer la zone de coupe pour l'insertion du guide et le retrait du couteau et, utilisée avec le guide, permet de vérifier la libération complète du ligament compressif.

De préférence, il existe un décrochement entre l'extrémité distale de la portion longitudinale du guide et le reste du canal, les parois latérales dudit canal étant plus basses que les parois latérales de l'extrémité distale. Ledit décrochement permet de crocheter le ligament à couper pour le bloquer dans le guide lorsque celui-ci est inséré sous ledit ligament.

On réalise ainsi un dispositif destiné à réaliser une libération d'un syndrome compressif de la main et notamment une libération du canal carpien, qui permet une protection entière des tissus mous environnants la zone de coupe et qui garantit une coupe sûre et efficace sans complexifier la technique opératoire.

## Revendications

1. Dispositif destiné à réaliser une libération d'un syndrome compressif de la main comprenant :
• un couteau (2) comprenant un manche (20) et une extrémité de coupe (22) recevant des moyens de coupe (23) ; et
• un guide (1 ;1') comprenant une portion longitudinale (11 ;11') avec une extrémité distale (110 ; 110') et une extrémité proximale (111 ; 111') et présentant au moins un canal (113 ; 113') dans lequel peut coulisser le couteau (2), ledit canal (113 ; 113') étant délimité par une première et une seconde parois latérales (1133a, 1133b ; 1133'a, 1133'b) dont les sommets (1136a, 1136b ; 1136'a, 1136'b) définissent un premier plan,
• le guide (1) comprenant en outre une portion transversale (12) s'étendant de part et d'autre de l'extrémité proximale (11), destinée à venir en appui sur une face essentiellement plane du bras ou de la main et définissant un second plan ;
**caractérisé par le fait que** le guide (1 ; 1') est agencé de sorte que, lors de l'insertion du guide (1 ; 1') et/ou une fois inséré dans sa position finale de service dans la zone à traiter de la main, le guide (1 ; 1') est dans une position dans laquelle le premier plan défini par les sommets (1136a, 1136b ; 1136'a, 1136'b) des première et seconde parois latérales (1133a, 1133b ; 1133'a, 1133'b) du canal (113 ; 113') forme un angle aigu non nul, de préférence compris entre 4° et 12°, avec le second plan défini par la portion transversale (12).

2. Dispositif selon la revendication 1, **caractérisé par le fait que** l'angle aigu non nul entre le premier plan et le second plan est ouvert en direction du nerf médian (4) lorsque le guide (1, 1') est en position finale de service dans la zone à traiter de la main.

3. Dispositif selon la revendication 1 ou 2, **caractérisé par le fait que** le premier plan défini par les sommets (1136a, 1136b) des première et seconde parois latérales (1133a, 1133b ; 1133'a, 1133'b) est parallèle au plan défini par le fond (1131) du canal (113).

4. Dispositif selon la revendication 3, **caractérisé par le fait que** la portion transversale (11) comprend des ailettes (121, 122) présentant une face supérieure (1211) essentiellement parallèle au premier plan et une face inférieure (1212) définissant le second plan et formant un angle aigu non nul, de préférence compris entre 4° et 12°, avec le premier plan, la face inférieure (1212) étant celle destinée à venir en appui sur la face antérieure de l'avant-bras.

5. Dispositif selon la revendication 1 ou 2, **caractérisé par le fait que** les première et une seconde parois latérales (1133'a, 1133'b) sont agencées de sorte que la première (1133'a) desdites parois latérales présente, sur la majeure partie de la longueur de la portion longitudinale (11') du guide (1'), une hauteur supérieure à la seconde paroi latérale (1133b') ; et de sorte que la différence de hauteur entre les première et seconde parois latérales (1133'a, 1133'b) est telle que le premier plan passant par le sommet de chacune desdites parois latérales (1133'a, 1133'b) forme un angle aigu non nul, de préférence compris entre 4° et 12°, avec le plan du fond (1131') du canal (113') et/ou avec le second plan défini par la portion transversale, lors de l'insertion du guide (1') et/ou une fois ledit guide (1') inséré dans sa position finale de service dans la zone à traiter de la main.

6. Dispositif selon l'une des revendications précédentes, **caractérisé par le fait que** l'extrémité distale (110 ; 110') de la portion longitudinale (11 ; 11') du guide (1 ; 1') présente essentiellement une forme d'ogive avec une pointe arrondie (1101) atraumatique et une partie courbée (1102) entre le fond (1131 ; 1131'), les première et seconde parois latérales (1133, 1134 ; 1133', 1134') du canal (113 ; 113') et ladite pointe (1101).

7. Dispositif selon l'une des revendications précédentes, **caractérisé par le fait que** ladite extrémité distale (110 ; 110') est agencée pour empêcher la translation du couteau (2) et de son extrémité de coupe (22) hors du canal (113 ; 113').

8. Dispositif selon l'une des revendications précédentes, **caractérisé par le fait que** l'extrémité distale (110 ; 110') a une forme d'ogive tronquée avec une partie supérieure plate (1103) pour permettre son identification par ultrasons.

9. Dispositif selon l'une des revendications précédentes, **caractérisé par le fait que** le couteau (2) comprend une languette inférieure (2212) destinée à coulisser contre le fond (1131 ; 1131') du canal (113 ; 113') et une languette supérieure (2211) destinée à faciliter l'insertion du couteau et à protéger les tissus environnants la zone de coupe, une encoche (221) entre lesdites languettes reçoit une lame de coupe (23) et forme l'extrémité de coupe (22) du couteau.

10. Dispositif selon la revendication précédente, **caractérisé par le fait que** la languette supérieure (2211) est agencée pour venir fermer l'extrémité distale (110 ; 110') du guide lorsque l'extrémité de coupe du couteau est en butée dans ladite extrémité distale.

11. Dispositif selon l'une des revendications précédentes, **caractérisé par le fait que** le couteau (2) et le guide (1 ; 1') sont agencés pour que ledit couteau (2) puisse pivoter dans le canal (113 ; 113') de la portion longitudinale (11 ; 11') du guide (1 ; 1') dans un sens ou dans l'autre, d'un angle compris de préférence entre 8° et 15°.

12. Dispositif selon l'une des revendications précédentes, **caractérisé par le fait qu'**il comprend en outre une spatule (3) comportant un manche (30) et une extrémité active (31) plate et arrondie ; et **par le fait que** la spatule (3) est agencée pour pouvoir coulisser dans le canal (113 ; 113') du guide jusqu'à venir en butée contre l'extrémité distale (110 ; 110') de la portion longitudinale (11 ; 11') du guide (1 ; 1').

13. Dispositif selon l'une des revendications précédentes, **caractérisé par le fait que** le canal (113 ; 113') présente à son extrémité distale des parois latérales hautes (1134 ; 1134') dont la hauteur est plus élevées que celles des première et seconde parois latérales (1133 ; 1133'), de sorte qu'il existe un décrochement (1135 ; 1135') entre l'extrémité distale (110 ; 110') de la portion longitudinale (11 ; 11') du guide (1 ; 1') et le reste du canal (113 ; 113'), ledit décrochement (1135 ; 1135') permettant de crocheter le ligament (6) à couper pour le bloquer dans le guide (1 ; 1') lorsque celui-ci est inséré sous ledit ligament.

## Patentansprüche

1. Vorrichtung zur Durchführung einer Dekompression eines Kompressionssyndroms der Hand, umfassend:
- ein Messer (2), das einen Griff (20) und ein Schneidende (22) umfasst, das Schneidmittel (23) aufnimmt; und
- eine Führung (1; 1'), die einen Längsabschnitt (11; 11') mit einem distalen Ende (110; 110') und einem proximalen Ende (111; 111') umfasst und mindestens einen Kanal (113; 113') aufweist, in dem das Messer (2) gleiten kann, wobei der Kanal (113; 113') von einer ersten und einer zweiten Seitenwand (1133a, 1133b; 1133'a, 1133'b) begrenzt ist, deren Spitzen (1136a, 1136b; 1136'a, 1136'b) eine erste Ebene definieren,
- wobei die Führung (1) ferner einen Querabschnitt (12) umfasst, der sich auf beiden Seiten des proximalen Endes (11) erstreckt und dazu bestimmt ist, gegen eine im Wesentlichen ebene Seite des Arms oder der Hand in Auflage zu gelangen, und eine zweite Ebene definiert;
**dadurch gekennzeichnet, dass** die Führung (1; 1') derart gestaltet ist, dass beim Einführen der Führung (1; 1') und/oder nachdem sie in ihre Betriebsendposition in der zu behandelnden Zone der Hand eingeführt wurde, die Führung (1; 1') sich in einer Position befindet, in der die erste Ebene, die von den Spitzen (1136a, 1136b; 1136'a, 1136'b) der ersten und der zweiten Seitenwand (1133a, 1133b; 1133'a, 1133'b) des Kanals (113; 113') definiert ist, einen von null verschiedenen spitzen Winkel, der vorzugsweise zwischen 4° und 12° beträgt, mit der zweiten Ebene bildet, die von dem Querabschnitt (12) definiert ist.

2. Vorrichtung nach Anspruch 1, **dadurch gekennzeichnet, dass** der von null verschiedene spitze Winkel zwischen der ersten Ebene und der zweiten Ebene in der Richtung des Mediannervs (4) offen ist, wenn die Führung (1, 1') sich in der Betriebsendposition in der zu behandelnden Zone der Hand befindet.

3. Vorrichtung nach Anspruch 1 oder 2, **dadurch gekennzeichnet, dass** die erste Ebene, die von den Spitzen (1136a, 1136b) der ersten und der zweiten Seitenwand (1133a, 1133b; 1133'a, 1133'b) definiert ist, parallel zu der Ebene ist, die von dem Boden (1131) des Kanals (113) definiert ist.

4. Vorrichtung nach Anspruch 3, **dadurch gekennzeichnet, dass** der Querabschnitt (11) Flügel (121, 122) umfasst, die eine obere Seite (1211), die im Wesentlichen parallel zur ersten Ebene ist, und eine untere Seite (1212) aufweisen, welche die zweite Ebene definiert und einen von null verschiedenen spitzen Winkel, der vorzugsweise zwischen 4° und 12° beträgt, mit der ersten Ebene bildet, wobei die untere Seite (1212) diejenige ist, die dazu bestimmt ist, auf der vorderen Seite des Vorderarms in Auflage zu gelangen.

5. Vorrichtung nach Anspruch 1 oder 2, **dadurch gekennzeichnet, dass** die erste und eine zweite Seitenwand (1133'a, 1133'b) derart gestaltet sind, dass die erste (1133'a) der Seitenwände auf dem Großteil der Länge des Längsabschnitts (11') der Führung (1') eine Höhe aufweist, die größer als die zweite Seitenwand (1133b') ist; und derart dass der Höhenunterschied zwischen der ersten und der zweiten Seitenwand (1133'a, 1133'b) derart ist, dass beim Einführen der Führung (1') und/oder nachdem die Führung (1') in ihre Betriebsendposition in der zu behandelnden Zone der Hand eingeführt wurde, die erste Ebene, die über die Spitze von jeder der Seitenwände (1133'a, 1133'b) verläuft, einen von null verschiedenen spitzen Winkel, der vorzugsweise zwischen 4° und 12° beträgt, mit der Ebene des Bodens (1131') des Kanals (113') und/oder mit der zweiten Ebene bildet, die von dem Querabschnitt definiert ist.

6. Vorrichtung nach einem der vorhergehenden Ansprüche, **dadurch gekennzeichnet, dass** das distale Ende (110; 110') des Längsabschnitts (11; 11') der Führung (1; 1') im Wesentlichen eine Spitzbogenform mit einer atraumatischen abgerundeten Spitze (1101) und einem gekrümmten Teil (1102) zwischen dem Boden (1131; 1131'), der ersten und der zweiten Seitenwand (1133, 1134; 1133'; 1134') des Kanals (113; 113') und der Spitze (1101) aufweist.

7. Vorrichtung nach einem der vorhergehenden Ansprüche, **dadurch gekennzeichnet, dass** das distale Ende (110; 110') dazu gestaltet ist, die Translation des Messers (2) und seines Schneidendes (22) aus dem Kanal (113; 113') zu verhindern.

8. Vorrichtung nach einem der vorhergehenden Ansprüche, **dadurch gekennzeichnet, dass** das distale Ende (110; 110') eine abgestumpfte Spitzbogenform mit einem flachen oberen Teil (1103) aufweist, um seine Identifikation per Ultraschall zu erlauben.

9. Vorrichtung nach einem der vorhergehenden Ansprüche, **dadurch gekennzeichnet, dass** das Messer (2) eine untere Zunge (2212), die dazu bestimmt ist, gegen den Boden (1131; 1131') des Kanals (113, 113') zu gleiten, und eine obere Zunge (2211) umfasst, die dazu bestimmt ist, die Einführung des Messers zu erleichtern und die umgebenden Gewebe der Schneidzone zu schützen, wobei eine Nut (221) zwischen den Zungen eine Schneidklinge (23) aufnimmt und das Schneidende (22) des Messers bildet.

10. Vorrichtung nach dem vorhergehenden Anspruch, **dadurch gekennzeichnet, dass** die obere Zunge (2211) dazu gestaltet ist, das distale Ende (110; 110') der Führung zu schließen, wenn das Schneidende des Messers in dem distalen Ende in Anschlag ist.

11. Vorrichtung nach einem der vorhergehenden Ansprüche, **dadurch gekennzeichnet, dass** das Messer (2) und die Führung (1; 1') so gestaltet sind, dass das Messer (2) in dem Kanal (113; 113') von dem Längsabschnitt (11; 11') der Führung (1; 1') in der einen oder der anderen Richtung um einen Winkel schwenken kann, der vorzugsweise zwischen 8° und 15° beträgt.

12. Vorrichtung nach einem der vorhergehenden Ansprüche, **dadurch gekennzeichnet, dass** sie ferner eine Spachtel (3) umfasst, die einen Griff (30) und ein flaches und abgerundetes aktives Ende (31) umfasst; und dadurch, dass die Spachtel (3) so gestaltet ist, dass sie in dem Kanal (113; 113') der Führung gleiten kann, bis sie gegen das distale Ende (110; 110') des Längsabschnitts (11; 11') der Führung (1; 1') in Anschlag gelangt.

13. Vorrichtung nach einem der vorhergehenden Ansprüche, **dadurch gekennzeichnet, dass** der Kanal (113; 113') an seinem distalen Ende hohe Seitenwände (1134; 1134') aufweist, deren Höhe größer als diejenigen der ersten und der zweiten Seitenwand (1133; 1133') ist, derart dass ein Versatz (1135; 1135') zwischen dem distalen Ende (110; 110') des Längsabschnitts (11; 11') der Führung (1; 1') und dem Rest des Kanals (113; 113') vorhanden ist, wobei der Versatz (1135; 1135') das Einhaken des zu schneidenden Bandes (6) erlaubt, um es in der Führung (1; 1') zu blockieren, wenn diese unter dem Band eingeführt ist.

## Claims

1. Device intended to effect a release of a compressive syndrome of the hand comprising:
• a scalpel (2) comprising a handle (20) and a cutting end (22) receiving cutting means (23); and
• a guide (1; 1') comprising a longitudinal portion (11; 11') with a distal end (110; 110') and a proximal end (111; 111') and having at least one channel (113; 113') in which the scalpel (2) can slide, said channel (113; 113') being defined by a first and a second side wall (1133a, 1133b; 1133'a, 1133'b), the tops (1136a, 1136b; 1136'a, 1136'b) of which define a first plane,
• the guide (1) further comprising a transverse portion (12) extending on both sides of the proximal end (11), intended to come into abutment on an essentially planar face of the arm or of the hand and defining a second plane;
**characterised in that** the guide (1; 1') is arranged so that, during the insertion of the guide (1; 1') and/or once inserted into its final usage position in the zone of the hand to be treated, the guide (1; 1') is in a position in which the first plane defined by the tops (1136a, 1136b; 1136'a, 1136'b) of the first and second side walls (1133a, 1133b; 1133'a, 1133'b) of the channel (113; 113') forms a non-zero acute angle, preferably between 4° and 12°, with the second plane defined by the transverse portion (12).

2. Device as claimed in claim 1, **characterised in that** the non-zero acute angle between the first plane and the second plane is open in the direction of the median nerve (4) when the guide (1, 1') is in the final usage position in the zone of the hand to be treated.

3. Device as claimed in claim 1 or 2, **characterised in that** the first plane defined by the tops (1136a, 1136b) of the first and second side walls (1133a, 1133b; 1133'a, 1133'b) is parallel to the plane defined by the bottom (1131) of the channel (113).

4. Device as claimed in claim 3, **characterised in that** the transverse portion (11) comprises wings (121, 122) having an upper face (1211) essentially parallel to the first plane and a lower face (1212) defining the second plane and forming a non-zero acute angle, preferably between 4° and 12°, with the first plane, the lower face (1212) being that intended to come into abutment on the anterior face of the forearm.

5. Device as claimed in claim 1 or 2, **characterised in that** the first and second side walls (1133'a, 1133'b) are arranged so that the first (1133'a) of said side walls has, over the major part of the length of the longitudinal portion (11') of the guide (1'), a height greater than the second side wall (1133b'); and so that the difference in height between the first and second side walls (1133'a, 1133'b) is such that the first plane passing across the top of each of said side walls (1133'a, 1133'b) forms a non-zero acute angle, preferably between 4° and 12°, with the plane of the bottom (1131') of the channel (113') and/or with the second plane defined by the transverse portion, during the insertion of the guide (1') and/or once said guide (1') is inserted into its final usage position in the zone of the hand to be treated.

6. Device as claimed in any one of the preceding claims **characterised in that** the distal end (110; 110') of the longitudinal portion (11; 11') of the guide (1; 1') is essentially in the shape of an ogive with an atraumatic rounded point (1101) and a curved part (1102) between the bottom (1131; 1131'), the first and second side walls (1133, 1134; 1133', 1134') of the channel (113; 113') and said point (1101).

7. Device as claimed in any one of the preceding claims, **characterised in that** said distal end (110; 110') is arranged to prevent the translation of the scalpel (2) and of its cutting end (22) out of the channel (113; 113').

8. Device as claimed in any one of the preceding claims, **characterised in that** the distal end (110; 110') is in the shape of a truncated ogive with a flat upper part (1103) to enable it to be identified by ultrasound.

9. Device as claimed in any one of the preceding claims, **characterised in that** the scalpel (2) comprises a lower tongue (2212) intended to slide against the bottom (1131; 1131') of the channel (113; 113') and an upper tongue (2211) intended to facilitate the insertion of the scalpel and to protect the tissues surrounding the cutting zone, a notch (221) between said tongues receives a cutting blade (23) and forms the cutting end (22) of the scalpel.

10. Device as claimed in the preceding claim, **characterised in that** the upper tongue (2211) is arranged to close the distal end (110; 110') of the guide when the cutting end of the scalpel is in abutment in said distal end.

11. Device as claimed in any one of the preceding claims, **characterised in that** the scalpel (2) and the guide (1; 1') are arranged so that said scalpel (2) can pivot in the channel (113; 113') of the longitudinal portion (11; 11') of the guide (1; 1') in one direction or in the other, by an angle preferably between 8° and 15°.

12. Device as claimed in any one of the preceding claims, **characterised in that** it further comprises a spatula (3) comprising a handle (30) and a flat, rounded active end (31); and **in that** the spatula (3) is arranged to be able to slide in the channel (113; 113') of the guide until coming into abutment against the distal end (110; 110') of the longitudinal portion (11; 11') of the guide (1; 1').

13. Device as claimed in any one of the preceding claims **characterised in that** the channel (113; 113') has, at its distal end, high side walls (1134; 1134'), the height of which is higher than those of the first and second side walls (1133; 1133'), so that there is a drop (1135; 1135') between the distal end (110; 110') of the longitudinal portion (11; 11') of the guide (1; 1') and the rest of the channel (113; 113'), said drop (1135; 1135') making it possible to hook the ligament (6) to be cut in order to immobilise it in the guide (1; 1') when the guide is inserted under said ligament.
